**Europäisches Patentamt**

(19) **European Patent Office**

**Office européen des brevets**

(11) Publication number : **0 209 192 B1**

(12) # EUROPEAN PATENT SPECIFICATION

(45) Date of publication of patent specification :
**05.06.91 Bulletin 91/23**

(51) Int. Cl.⁵ : **A61L 2/18**, A01N 57/10

(21) Application number : **86201215.0**

(22) Date of filing : **11.07.86**

(54) **Contact-lens liquid.**

(30) Priority : **11.07.85 NL 8502002**

(43) Date of publication of application :
**21.01.87 Bulletin 87/04**

(45) Publication of the grant of the patent :
**05.06.91 Bulletin 91/23**

(84) Designated Contracting States :
**AT BE CH DE FR GB IT LI LU NL SE**

(56) References cited :
**EP-A- 0 095 524**
**DE-A- 1 921 358**
**FR-A- 2 195 802**
**US-A- 4 029 817**
**US-A- 4 209 449**

(73) Proprietor : **ICN PHARMACEUTICALS HOLLAND B.V.**
**P.O. Box 328**
**NL-2700 AH Zoetermeer (NL)**

(72) Inventor : **Van Doorne, Hans**
**De Hulst 15**
**NL-9301 PB Roden (NL)**
Inventor : **Spruijt, Adrianus Franciscus**
**Albrechtsveld 67**
**NL-2804 WD Gouda (NL)**

(74) Representative : **van der Beek, George Frans, Ir. et al**
**Nederlandsch Octrooibureau**
**Scheveningseweg 82 P.O. Box 29720**
**NL-2502 LS 's-Gravenhage (NL)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The invention relates to a contact-lens liquid which contains

a) a surface-active compound,
b) sodium chloride,
c) buffering substances,
d) EDTA,
e) a preservative and, if desired,
f) a thickening agent.

Such contact-lens liquids are generally known.

In the case of contact-lens liquids a distinction can be made between the so-called multiple solutions and the "all-in-one solution". In the case of the multiple solution different solutions are used for introducing the contact-lens into the eye and protecting the lens of the eye, while in the case of the all-in-one solution the same solution is used.

A single solution is described in US Patent 4,126,587 in which a mixture of fatty acid amides is described.

US Patent 4,048,122 also describes a cleaning liquid for soft and silicone contact-lenses in which use is made of the same fatty acid as described above, together with a water-soluble polyalkyleneoxy-modified dimethylsilicone resin.

Contact-lens liquids are solutions which are intended to make a prolonged and comfortable use of lenses possible. Because the liquid may come/comes into direct contact with one of the most sensitive organs of the human body (the eye), they have to be sterile products. Two alternatives may be considered for guaranteeing the sterility during use. Firstly, the solutions may be supplied in so-called single-dose units. However, this is an expensive option. Secondly, a suitable compound with antimicrobial activity may be added to the product. The vast majority of manufacturers of contact-lens liquids have chosen this solution.

The manufacturer is subject to certain limitations with regard to the choice of the preservative to be used.

The finished contact-lens liquid must meet certain minimum requirements with regard to the antimicrobial action. The following requirements may be imposed for a so-called all-in-one liquid, i.e. a product which is intended for disinfecting, cleaning, preserving and inserting the lenses :

– for bacteria, a reduction of the number of live nuclei by at least a factor of 1000 must be achieved in a period of six hours. In the specialist literature this is often expressed in two other ways, viz. reduction by at least three powers of ten or a reduction by three logarithmic cycles must be achieved.

– for yeasts and moulds a reduction of at least a factor of 100 (a reduction by two powers of ten or two logarithmic cycles) must be achieved within a period of two weeks.

Drawbacks were associated with preservatives used hitherto.

It has now been found that a so-called all-in-one liquid can be prepared which produces no eye irritation but is quite stable and it leads to coating of the prescription by including as preservative a quaternary phosphorus compound having the formula 1 given on the formula sheet, wherein

R represents an amido amino radical having the formula 2, wherein the formula 2

$R^1$ represents an alkyl, alkenyl, alkoxy or hydroxyalkyl group having 5-22 carbon atoms or an aryl or alkaryl group of at most 20 carbon atoms,

$R^2$ represents a hydrogen atom or alkyl, hydroxyalkyl or alkenyl group with at most 6 carbon atoms each or a cycloalkyl group having at most 6 carbon atoms or a polyoxyalkylene group with at most 10 carbon atoms,

$R^3$ and $R^4$, which may or may not be identical, represent an alkyl, hydroxyalkyl or carboxyalkyl group of at most 6 carbon atoms in each alkyl radical or a polyoxyalkylene group of at most 10 carbon atoms, where $R^3$ and $R^4$, together with the nitrogen atom to which they are bound, may represent a heterocyclic nitrogen-containing ring, and n is an integer from 2 to 12 and X represents an anion.

Preferably a compound having the formula 3 is used in which R represents an alkyl radical having 5-17 carbon atoms.

The compounds having the formulae 1 to 3 are already known and in particular, from the US Patent 4,209,449, in which it is reported that they have excellent foaming, viscosity-enhancing, wetting, cleansing, detergent, antistatic-promoting, emulsifying and bacterio-static properties. These very stable compounds are tolerated well by human tissue and exhibit a low oral toxicity and low eye irritation. It is indicated that as a result they are outstandingly suitable for use for cosmetic purposes as well as for use in industry.

For use in a so-called all-in-one liquid the requirements to be imposed are, however, much higher and the requirements imposed above must be achieved. That of the large number of possible compounds it was precisely the present compound which gave much better results was not to be expected.

Expediently the composition contains component a) in a concentration of at most 10% by weight, preferably at most 5% by weight. A suitable component a) is dipluronic (poloxamer). This is preferably used in a quantity of at most 1.0% by weight of dipluronic, very particularly 0.2-0.6% by weight of dipluronic. The sodium chloride is expediently present in a concentration of at most 2% by weight. As a buffering substance, preferably at most 17% by weight of borax (disodium borate) together with at most 10% by weight of boric acid, is used. As EDTA. preferably disodium EDTA is used in a concentration of at most 3% by weight. A content of 0.5% by weight of disodium EDTA is preferred.

The content of sodium chloride is preferably at most 0.5% by weight, while the content of boric acid is preferably 0.5-2.5% by weight. The content of sodium borate is preferably 0.05-5% by weight, in particular 0.01-0.8% by weight.

Particularly good results are obtained if at least 0.0001% by weight and at most 0.06% by weight of thiomersal (sodium ethylmercurithiosalicylate) is also present.

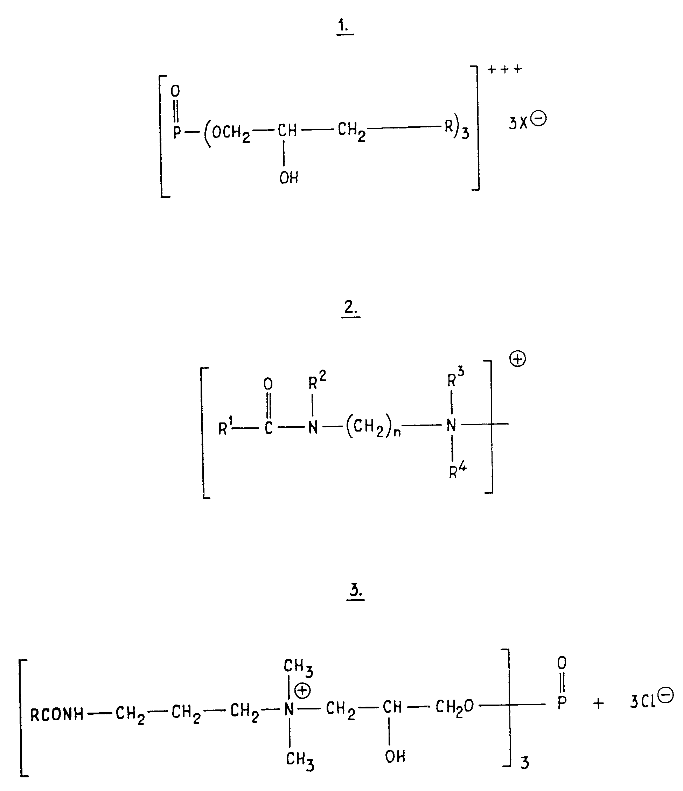

**Claims**

1. Contact-lens liquid which contains
a) a surface-active compound,
b) sodium chloride,
c) buffering substances,
d) EDTA,
e) a preservative and, if desired,
f) a thickening agent, characterized in that it contains as preservative a quaternary phosphorus compound having the formula 1 wherein
R represents an amido amine radical having the formula 2 wherein the formula 2
$R^1$ represents an alkyl, alkenyl, alkoxy or hydroxyalkyl group having 5-22 carbon atoms, or an aryl or alkaryl group of at most 20 carbon atoms,
$R^2$ represents a hydrogen atom or alkyl, hydroxyalkyl, or alkenyl group having at most 6 carbon atoms each or a cycloalkyl group having at most 6 carbon atoms or a polyoxyalkylene group having at most 10 carbon atoms,
$R^3$ and $R^4$, which may or may not be identical, represent an alkyl, hydroxyalkyl, carboxyalkyl group of at most 6 carbon atoms in each alkyl radical or a polyoxyalkylene group of at most 10 carbon atoms, where $R^3$ and $R^4$, together with the nitrogen atom to which they are bound, may represent a heterocyclic nitrogencontaining ring, and n is an integer from 2 to 12 and X represents an anion.

2. Contact-lens liquid according to Claim 1, characterized in that as preservative a compound having the formula 3 is used, where R represents an alkyl radical having 5-17 carbon atoms.

3. Contact-lens liquid according to Claim 1, characterized in that it contains component a) in a concentration of at most 10% by weight.

4. Contact-lens liquid according to Claims 1-3, characterized in that it contains at most 5% by weight of component a).

5. Contact-lens liquid according to Claims 1-4, characterized in that it contains sodium chloride in a concentration of at most 2% by weight.

6. Contact-lens liquid according to Claims 1-8, characterized in that it contains at most 0.5% by weight of sodium chloride.

7. Contact-lens liquid according to Claims 1-6, characterized in that it contains at most 17% by weight of borax (disodium borate), together with at most 10% by weight of boric acid, as buffering substance.

8. Contact-lens liquid according to Claims 1-7, characterized in that it contains at most 3% by weight of EDTA, preferably disodium EDTA.

9. Contact-lens liquid according to Claim 7, characterized in that the EDTA is disodium EDTA.

10. Contact-lens liquid according to Claims 1-7, characterized in that it contains 0.1% by weight of disodium EDTA.

11. Contact-lens liquid according to Claims 1-8, characterized in that it contains at least 0.0001% by weight and at most 0.06% by weight of thiomersal (sodium ethylmercurithiosalicylate).

12. Contact-lens liquid according to Claims 1-11, characterized in that it contains at most 1.0% by weight of dipluronic as a component.

13. Contact-lens liquid according to Claim 12, characterized in that it contains 0.2-0.6% by weight of dipluronic.

14. Contact-lens liquid according to Claims 1-13, characterized in that it contains 0.5-2.5% by weight of boric acid.

15. Contact-lens liquid according to Claims 1-14, characterized in that it contains 0.05-5% by weight of sodium borate.

16. Contact-lens liquid according to Claims 1-15, characterized in that it contains 0.01-0.8% by weight of sodium borate.

**Ansprüche**

1. Kontaktlinsenflüssigkeit, die
a) eine oberflächenaktive Verbindung,
b) Natriumchlorid,
c) Puffersubstanzen,
d) EDTA,
e) ein Konservierungsmittel und, wenn gewünscht,
f) ein Verdickungsmittel enthält, dadurch **gekennzeichnet**, daß sie als Konservierungsmittel eine quaternäre Phosphorverbindung mit der Formel 1 enthält, worin
R ein Amidoaminrest mit der Formel 2 bedeutet, worin in der Formel 2 $R^1$ eine Alkyl-, Alkenyl-, Alkoxy-oder Hydroxyalkylgruppe mit 5 bis 22 Kohlenstoffatomen oder eine Aryl- oder Alkarylgruppe mit höchstens 20 Kohlenstoffatomen bedeutet,
$R^2$ ein Wasserstoffatom oder eine Alkyl-, Hydroxyalkyl- oder Alkenylgruppe mit jeweils höchstens 6 Kohlenstoffatomen oder eine Cycloalkylgruppe mit höchstens 6 Kohlenstoffatomen oder eine Polyoxyalkylengruppe mit höchstens 10 Kohlenstoffatomen bedeutet,

R³ und R⁴, die gleich oder verschieden sein können, eine Alkyl-, Hydroxyalkyl-, Carboxyalkylgruppe mit höchstens 6 Kohlenstoffatomen in jedem Alkylrest oder eine Polyoxyalkylengruppe mit höchstens 10 Kohlenstoffatomen bedeuten, worin R³ und R⁴ zusammen mit dem Stickstoffatom, an das sie gebunden sind, einen heterocyclischen, stickstoffhaltigen Ring bedeuten können, und

n eine ganze Zahl von 2 bis 12 ist und
X ein Anion bedeutet.

2. Kontaktlinsenflüssigkeit nach Anspruch 1, dadurch gekennzeichnet, daß als Konservierungsmittel eine Verbindung mit der Formel 3 verwendet wird, worin R einen Alkylrest mit 5 bis 17 Kohlenstoffatomen bedeutet.

3. Kontaktlinsenflüssigkeit nach Anspruch 1, dadurch gekennzeichnet, daß sie die Komponente a) in einer Konzentration von höchstens 10 Gew.-% enthält.

4. Kontaktlinsenflüssigkeit nach den Ansprüchen 1 bis 3, dadurch gekennzeichnet, daß sie höchstens 5 Gew.-% der Komponente a) enthält.

5. Kontaktlinsenflüssigkeit nach den Ansprüchen 1 bis 4, dadurch gekennzeichnet, daß sie Natriumchlorid in einer Konzentration von höchstens 2 Gew.-% enthält.

6. Kontaktlinsenflüssigkeit nach den Ansprüchen 1 bis 5, dadurch gekennzeichnet, daß sie höchstens 0,5 Gew.-% Natriumchlorid enthält.

7. Kontaktlinsenflüssigkeit nach den Ansprüchen 1 bis 6, dadurch gekennzeichnet, daß sie höchstens 17 Gew.-% Borax (Dinatriumborat) zusammen mit höchstens 10 Gew.-% Borsäure als Puffersubstanz enthält.

8. Kontaktlinsenflüssigkeit nach den Ansprüchen 1 bis 7, dadurch gekennzeichnet, daß sie höchstens 3 Gew.-% EDTA, vorzugsweise Dinatrium-EDTA, enthält.

9. Kontaktlinsenflüssigkeit nach Anspruch 7, dadurch gekennzeichnet, daß das EDTA Dinatrium-EDTA ist.

10. Kontaktlinsenflüssigkeit nach den Ansprüchen 1 bis 7, dadurch gekennzeichnet, daß sie 0,1 Gew.-% Dinatrium-EDTA enthält.

11. Kontaktlinsenflüssigkeit nach den Ansprüchen 1 bis 8, dadurch gekennzeichnet, daß sie wenigstens 0,001 Gew.-% und höchstens 0,06 Gew.-% Thiomersal (Natrium-ethylquecksilberthiosalicylat) enthält.

12. Kontaktlinsenflüssigkeit nach den Ansprüchen 1 bis 11, dadurch gekennzeichnet, daß sie höchstens 1 Gew.-% Dipluronic als Komponente enthält.

13. Kontaktlinsenflüssigkeit nach Anspruch 12, dadurch gekennzeichnet, daß sie 0,2 bis 0,6 Gew.-% Dipluronic enthält.

14. Kontaktlinsenflüssigkeit nach den Ansprüchen 1 bis 13, dadurch gekennzeichnet, daß sie 0,5 bis 2,5 Gew.-% Borsäure enthält.

15. Kontaktlinsenflüssigkeit nach den Ansprüchen 1 bis 14, dadurch gekennzeichnet, daß sie 0,05 bis 5 Gew.-% Natriumborat enthält.

16. Kontaktlinsenflüssigkeit nach den Ansprüchen 1 bis 15, dadurch gekennzeichnet, daß sie 0,01 bis 0,8 Gew.-% Natriumborat enthält.

## Revendications

1. Liquide pour lentilles de contact comprenant :
a) un composé tensio-actif,
b) du chlorure de sodium,
c) des substances tampon,
d) de l'EDTA,
e) un conservateur et, si nécessaire,
f) un agent épaississant,
caractérisé en ce que, comme conservateur, le liquide comprend un composé phosphoré quaternaire de formule 1 dans laquelle :
R représente un radical amido amine de formule 2 dans laquelle :
R¹ représente un groupe alcoyle, alcényle, alcoxy ou hydroxyalcoyle ayant de 5 à 22 atomes de carbone ou un groupe aryle ou alcaryle d'au plus 20 atomes de carbone,
R² représente un atome d'hydrogène ou un groupe alcoyle, hydroxyalcoyle ou alcényle ayant chacun au plus 6 atomes de carbone ou un groupe cycloalcoyle ayant au plus 6 atomes de carbone ou un groupe polyoxyalcoylène ayant au plus 10 atomes de carbone,
R³ et R⁴, qui peuvent être identiques ou différents, représentent un groupe alcoyle, hydroxyalcoyle, carboxyalcoyle dont le radical alcoyle respectif comprend au plus 6 atomes de carbone ou un groupe polyoxyalcoylène ayant au plus 10 atomes de carbone, où R³ et R⁴, avec l'atome d'azote auquel ils sont liés, peuvent représenter un noyau hétérocyclique contenant de l'azote et n est un nombre entier de 2 à 12 et X représente un anion.

2. Liquide pour lentilles de contact selon la revendication 1, caractérisé en ce que, comme conservateur, on utilise un composé de formule 3 dans laquelle R représente un radical alcoyle ayant de 5 à 17 atomes de carbone.

3. Liquide pour lentilles de contact selon la revendication 1, caractérisé en ce qu'il comprend le composant a) en concentration d'au plus 10% en poids.

4. Liquide pour lentilles de contact selon les revendications 1 à 3, caractérisé en ce qu'il comprend au plus 5% en poids du composant a).

5. Liquide pour lentilles de contact selon les revendications 1 à 4, caractérisé en ce qu'il comprend du chlorure de sodium en concentration d'au plus 2% en poids.

6. Liquide pour lentilles de contact selon les revendications 1 à 8, caractérisé en ce qu'il comprend au plus 0,5% en poids de chlorure de sodium.

7. Liquide pour lentilles de contact selon les revendications 1 à 6, caractérisé en ce qu'il comprend au plus 17% en poids de borax (borate disodique) avec au plus 10% en poids d'acide borique comme substance tampon.

8. Liquide pour lentilles de contact selon les revendications 1 à 7, caractérisé en ce qu'il comprend au plus 3% en poids d'EDTA, de préférence d'EDTA disodique.

9. Liquide pour lentilles de contact selon la revendication 7, caractérisé en ce que l'EDTA est de l'EDTA disodique.

10. Liquide pour lentilles de contact selon les revendications 1 à 7, caractérisé en ce qu'il comprend 0,1% en poids d'EDTA disodique.

11. Liquide pour lentilles de contact selon les revendications 1 à 8, caractérisé en ce qu'il comprend au moins 0,0001% en poids et au plus 0,06% en poids de thiomersal (sodium éthylmercurithiosalicylate).

12. Liquide pour lentilles de contact selon les revendications 1 à 11, caractérisé en ce qu'il comprend au plus 1,0% en poids de dipluronique comme composant.

13. Liquide pour lentilles de contact selon la revendication 12, caractérisé en ce qu'il comprend de 0,2 à 0,6% en poids de dipluronique.

14. Liquide pour lentilles de contact selon les revendications 1 à 13, caractérisé en ce qu'il comprend de 0,5% à 2,5% en poids d'acide borique.

15. Liquide pour lentilles de contact selon les revendications 1 à 14, caractérisé en ce qu'il comprend de 0,05 à 5% en poids de borate de sodium.

16. Liquide pour lentilles de contact selon les revendications 1 à 15, caractérisé en ce qu'il comprend de 0,01 à 0,8% en poids de borate de sodium.

1.

$$\left[ \begin{array}{c} O \\ \parallel \\ P-(OCH_2-CH-CH_2-R)_3 \\ \qquad\qquad | \\ \qquad\qquad OH \end{array} \right]^{+++} 3X^{\ominus}$$

2.

$$\left[ \begin{array}{c} O \qquad R^2 \qquad\qquad R^3 \\ \parallel \qquad | \qquad\qquad | \\ R^1-C-N-(CH_2)_n-N- \\ \qquad\qquad\qquad\qquad\qquad | \\ \qquad\qquad\qquad\qquad\qquad R^4 \end{array} \right]^{\oplus}$$

3.

$$\left[ \begin{array}{c} CH_3 \\ | \oplus \\ RCONH-CH_2-CH_2-CH_2-N-CH_2-CH-CH_2O- \\ \qquad\qquad\qquad\qquad\qquad | \qquad\qquad\qquad | \\ \qquad\qquad\qquad\qquad\qquad CH_3 \qquad\qquad OH \end{array} \right]_3 \begin{array}{c} O \\ \parallel \\ P \end{array} + 3Cl^{\ominus}$$